(19) Europäisches Patentamt
European Patent Office
Office européen des brevets

(11) Veröffentlichungsnummer : **0 161 544**
**B1**

(12) # EUROPÄISCHE PATENTSCHRIFT

(45) Veröffentlichungstag der Patentschrift : **02.03.88**

(51) Int. Cl.[4] : **C 07 C 53/02**, C 07 C 51/09, C 07 C 27/02

(21) Anmeldenummer : **85104902.3**

(22) Anmeldetag : **23.04.85**

(54) **Verfahren zur Herstellung von Ameisensäure.**

(30) Priorität : **14.05.84 DE 3417790**

(43) Veröffentlichungstag der Anmeldung : **21.11.85 Patentblatt 85/47**

(45) Bekanntmachung des Hinweises auf die Patenterteilung : **02.03.88 Patentblatt 88/09**

(84) Benannte Vertragsstaaten : **AT BE DE FR GB IT NL SE**

(56) Entgegenhaltungen :
**EP-A- 0 012 321**
**EP-A- 0 017 866**
**EP-A- 0 107 441**
**DE-A- 2 545 730**

(73) Patentinhaber : **BASF Aktiengesellschaft**
**Carl-Bosch-Strasse 38**
**D-6700 Ludwigshafen (DE)**

(72) Erfinder : **Hohenschutz, Heinz, Dr.**
**Leibnizstrasse 12**
**D-6800 Mannheim 1 (DE)**
Erfinder : **Schmidt, Johannes E., Dr.**
**Pariser Strasse 23**
**D-6700 Ludwigshafen (DE)**
Erfinder : **Kiefer, Hans, Dr.**
**Im Sandgarten 5**
**D-6706 Wachenheim (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 161 544 B1

**Beschreibung**

Die vorliegende Erfindung betrifft ein neues Verfahren zur Herstellung von Ameisensäure durch Hydrolyse von Methylformiat und anschließende Gewinnung einer wasserfreien oder weitgehend wasserfreien Säure durch destillative Zerlegung des Hydrolysegemisches.

Dieses grundlegende Verfahren ist allgemein bekannt, vermag aber nicht zu befriedigen, weil sich das Hydrolysegleichgewicht

$$H—CO—O—CH_3 + H_2O \rightleftharpoons H—COOH + CH_3OH$$

nur sehr langsam einstellt und weil es Schwierigkeiten bereitet, die Ameisensäure aus dem wäßrigen Hydrolysegemisch in wasserfreier oder weitgehend wasserfreier Form zu isolieren.

Die Lösung dieser Probleme ist Gegenstand zahlreicher Untersuchungen, die jedoch nur bei speziellen technischen Verfahrensausgestaltungen als erfolgreich oder bedingt erfolgreich bezeichnet werden können.

Nach der Lehre der EP-PS 00 01 432 nimmt man die Hydrolyse in Gegenwart von tertiären Stickstoffbasen vor, die wie bestimmte Imidazolderivate einen pKA-Wert von 4-9 haben. Hierdurch wird die Ameisensäure durch Adduktbildung mit der Base dem Gleichgewicht weitgehend entzogen, so daß man einerseits einen hohen Hydrolysegrad erzielen kann, jedoch verläuft die Hydrolyse andererseits wesentlich langsamer als ohne die Base. Weiterhin läßt sich das Wasser zwar ohne weiteres vollständig von dem salzartigen Addukt abtrennen, jedoch erfordert die Spaltung des Adduktes zur destillativen Gewinnung der Säure entweder so hohe Temperaturen, daß sich die Ameisensäure hierbei bereits merklich zersetzt, oder eine unwirtschaftlich starke Verminderung des Druckes.

Das Problem der Gewinnung der wasserfreien Säure aus dem Hydrolysegemisch wird durch das Verfahren der DE-OS 25 45 658 hervorragend gelöst, bei welchem wasserunlösliche Formamide, insbesondere Di-n-butylformamid, als Extraktionsmittel dienen. Auch hierbei bildet sich ein Addukt aus dem Formamid und der Säure, jedoch läßt sich die Säure hiervon bequem abdestillieren, ohne daß sie sich merklich zersetzt.

Technisch kontinuierliche Ausgestaltungen dieses grundlegenden Verfahrens sind Gegenstand der EP-PS 0 012 321 und der EP-PS 0 017 866.

Die erstgenannte Patentschrift betrifft die Durchführung der Hydrolyse im Mittelteil einer Destillationskolonne, in dessen unterem Bereich eines der wasserunlöslichen Formamide zugegen ist, welches die entstehende Säure bindet und in Form eines relativ hochsiedenden Adduktes in den unteren Teil der Kolonne gelangen läßt. Die so erhaltene Sumpffraktion wird dann in einer anschließenden Destillationskolonne in Säure und Formamid zerlegt, wonach das Formamid wieder in den unteren Bereich des Hydrolyseteils der Kolonne zurückgeführt wird.

Das Verfahren der EP-PS 0 017 866 beruht im wesentlichen darauf, die Ameisensäure mit Hilfe der wasserunlöslichen Formamide nach der Methodik der Flüssig-flüssig-Extraktion aus dem Hydrolysegemisch zu gewinnen und ist technisch entsprechend hiernach ausgerichtet. In einer besonderen Ausführungsform dieses Verfahrens wird die Mitverwendung der wasserunlöslichen Formamide bei der Hydrolyse empfohlen. Dies wirkt sich wegen der Adduktbildung zwar günstig auf den Hydrolysegrad aus, ist verfahrenstechnisch aber insofern nachteilig, als man bei der destillativen Abtrennung des überschüssigen Methylformiates und des Methanols vom Hydrolysegemisch eine zweiphasige Sumpffraktion aus wäßriger Ameisensäure einerseits und Formamid/Ameisensäure andererseits erhält, die zunächst zu trennen wäre, da sie andernfalls den harmonischen Ablauf der kontinuierlichen Flüssig-flüssig-Extraktion stören würde. Es hat sich daher als zweckmäßiger erwiesen, die Hydrolyse nicht durch die wasserunlöslichen Formamide sondern durch einen großen Wasserüberschuß zu begünstigen, zumal im gesamten Verfahren kein Wasser verdampft werden muß und selbst ein großer Überschuß im Hinblick auf den Energieverbrauch praktisch zu vernachlässigen ist.

So sehr sich das Verfahren der EP-PS 0 017 866 insgesamt auch empfiehlt, ist es jedoch insofern von begrenzter Anwendbarkeit, als es apparativ sehr aufwendig ist und als es deshalb wegen der hohen Investitionskosten nur ab einem bestimmten Grenzwert für die Produktionskapazität, der sich nach den jeweiligen wirtschaftlichen Gegebenheiten richtet, rentabel ist.

Schließlich ist es aus der DE-OS 25 45 730 bekannt, wäßrige Ameisensäure der Extraktivdestillation mit N-Formylmorpholin zu unterwerfen und von der hierbei anfallenden wasserfreien oder wasserarmen Sumpffraktion wasserfreie bzw. -arme Säure abzudestillieren.

Der Erfindung lag nun die Entwicklung eines neuen Verfahrens zur Herstellung von wasserfreier oder weitgehend wasserfreier Ameisensäure zugrunde, welches insbesondere für kleinere Produktionskapazitäten eine technische und wirtschaftliche Alternative zum Verfahren der EP-PS 0 017 866 bietet.

Demgemäß wurde ein neues Verfahren zur Herstellung von wasserfreier oder weitgehend wasserfreier Ameisensäure durch Hydrolyse von Methylformiat in Gegenwart eines Formamids und anschließende destillative Gewinnung der Ameisensäure aus dem Hydrolysegemisch gefunden, welches dadurch gekennzeichnet ist, daß man die Hydrolyse in Gegenwart von 0,5-3 mol pro Mol Methylformiat eines wasserlöslichen Formamides der allgemeinen Formel I

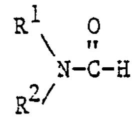

(I)

vornimmt, in welcher $R^1$ und $R^2$ Alkylgruppen oder gemeinsam unter Ausbildung eines 5- bis 7-gliedrigen Ringes eine Alkylengruppe mit der Maßgabe bedeuten, daß die Summe der C-Atome der Reste $R^1$ und $R^2$ 5 oder 6 beträgt und daß im Falle einer Alkylengruppe ein C-Atom, welches nicht unmittelbar mit dem N-Atom verbunden ist, durch ein Sauerstoffatom ersetzt sein kann.

Weiterhin wurde gefunden, daß die Hydrolyse besonders schnell und mit hohem Umsatz gelingt, wenn man sie in einer ersten Stufe ohne die Mitverwendung nennenswerter Mengen des Formamides I bis zur Gleichgewichtseinstellung führt und wenn man sie zur Erhöhung des Umsatzes in einer zweiten Stufe in Gegenwart der definitionsgemäßen Mengen eines Formamides I, bezogen auf die Menge des ursprünglich eingesetzten Methylformiates, vornimmt.

Bezüglich der beiden Funktionen, nämlich der Begünstigung der Hydrolyse unter möglichst geringem Wasserzusatz und der störungsfreien destillativen Gewinnung der Ameisensäure aus den Formamid/Ameisensäure-Addukten, weisen die Formamide I ein unerwartetes Wirkungsoptimum auf, wobei hinzuzufügen wäre, daß sie auch keine unerwünschten Nebenreaktionen eingehen und deshalb im Falle der technisch besonders wichtigen kontinuierlichen Verfahrensausgestaltung über lange Betriebszeiten hinweg in ihrem Stoffkreislauf praktisch unverändert bleiben.

Unter den Formamiden I haben das Di-n-propylformamid und das N-Formylmorpholin besonders große Bedeutung ; daneben kommt aber auch z. B. N-Formyl-piperidin in Betracht.

Allgemein beträgt das Molverhältnis von I zum eingesetzten Methylformiat 0,5 : 1 bis 3 : 1, vorzugsweise 1 : 1 bis 1,5 : 1. Nimmt man die Hydrolyse gemäß bevorzugter Ausführungsform zweistufig vor, wobei es sich empfiehlt, in der ersten Stufe gar kein Formamid I oder nur untergeordnete Mengen — etwa bis zu 10 % ihrer Gesamtmenge — mitzuverwenden, gelten die gleichen Molverhältnisse, da es weniger auf das noch vorhandene Methylformiat sondern vielmehr auf die entstandene Ameisensäure ankommt.

Da der Hydrolysegrad bis zu einem Wasserüberschuß von etwa 10 mol/mol Methylformiat merklich zunimmt — höhere Überschüsse bedingen keine nennenswerten Verbesserungen mehr — wäre es an sich zweckmäßig, das Wasser in einem solchen Überschuß einzusetzen, wenn es bei der Destillation nicht wieder unter hohem Energieverbrauch verdampft werden müßte. Die wirtschaftlich optimale Wassermengen liegen daher bei der erfindungsgemäßen Mitverwendung der Formamide I zwischen etwa 1 und 2 mol pro Mol Methylformiat, wobei bei der zweistufigen Hydrolyse das gesamte Wasser oder der größte Teil in den ersten Reaktor gegeben werden sollte.

Die Hydrolysetemperaturen liegen bei der einstufigen Hydrolyse zweckmäßigerweise zwischen 120 und 140 °C, und bei der zweistufigen Arbeitsweise in beiden Reaktoren zwischen 90 und 110 °C. Hieraus ergibt sich ein Druckbereich von ungefähr 5-15 bar.

Die Mitverwendung von Hydrolysekatalysatoren wie Schwefelsäure oder p-Toluolsulfonsäure ist möglich, bringt aber keine nennenswerten Vorteile, weil die entstehende Ameisensäure als starke Säure autokatalytisch wirkt.

Der nach dem erfindungsgemäßen Verfahren erzielbare Hydrolysegrad beträgt etwa 40-45 %, wobei die zweistufige Hydrolyse etwa zwei- bis viermal schneller verläuft als die einstufige.

Im Anschluß an die Hydrolyse trennt man überschüssiges Methylformiat und Methanol wie üblich durch Destillation ab und führt diese Stoffe zweckmäßigerweise in die Hydrolyse bzw. in die Stufe der Methylformiatsynthese zurück.

Als Sumpfprodukt dieser Destillation erhält man ein Gemisch aus Wasser, Ameisensäure und dem Formamid I, wobei die Säure in Form eines losen Adduktes mit dem Amid vorliegt. Von dieser Fraktion wird das Wasser in einer weiteren Kolonne wie üblich bei etwa 80-150 °C Sumpftemperatur und 400-1 000 mbar abdestilliert und in die Hydrolyse zurückgeführt. Möchte man keine reine, sondern nur hochkonzentrierte wäßrige Ameisensäure gewinnen, so läßt man entsprechende Mengen Wasser zurück.

Das Sumpfprodukt aus der letztgenannten Destillationsstufe wird sodann in einem weiteren Destillationsschritt ebenfalls in an sich bekannter Weise in eine Kopffraktion aus reiner oder konzentrierter Ameisensäure und eine Sumpffraktion aus dem Formamid I zerlegt, welche in die Hydrolysestufe zurückgeführt wird. Zweckmäßigerweise nimmt man diese Destillation bei 50-300 mbar und einer Sumpftemperatur von 120-170 °C vor.

Arbeitet man diskontinuierlich kann man die drei Destillationsschritte auch in zeitlicher Reihenfolge in der gleichen Kolonne vornehmen, und bei kontinuierlicher Betriebsweise ist es auch möglich, die ersten beiden Schritte in nur einer Kolonne unter Seitenabzug des Wassers vorzunehmen.

Beispiel 1

In einen Rührreaktor, der auf 140 °C Innentemperatur gehalten wurde und der unter einem Druck von rd. 8 bar stand, wurden

61,8 kg/h einer Lösung aus 60 kg (1 kmol/h) Methylformiat und 1,8 kg Methanol
18,0 kg/h (1 kmol/h) Wasser und
129,0 kg/h (rd. 1 kmol/h) Di-n-propylformamid

kontinuierlich eingeführt und bei einer mittleren Verweilzeit von 3 h miteinander umgesetzt. Der Reaktoraustrag, der aus

35,4 kg/h Methylformiat
14,9 kg/h Methanol
17,9 kg/h Ameisensäure
11,0 kg/h Wasser und
129,0 kg/h Di-n-propylformamid

bestand, wurde unter Entspannung auf den 12. Boden (von unten gezählt) einer Glockenbodenkolonne mit 35 Böden gegeben und kontinuierlich bei Normaldruck fraktioniert. Hierbei wurden bei einem Rücklaufverhältnis von 1 erhalten :

35,6 kg/h eines Gemisches aus 34,5 kg Methylformiat und 1,1 kg Methanol als Kopffraktion, welche in den Hydrolysereaktor zurückgeführt wurde
14,7 kg/h eines flüssigen, dem 28. Boden entnommenen Seitenabzugs aus 13,8 kg/h Methanol und 0,9 kg/h Methylformiat und
157,9 kg/h eines Gemisches aus 17,9 kg/h Ameisensäure, 11,0 kg/h Wasser und 129,0 kg/h Di-n-propylformamid als Sumpffraktion.

Die Sumpffraktion wurde in einer Füllkörperkolonne (15 theoretische Böden) bei Normaldruck wie üblich weitgehend entwässert, wobei als Sumpfprodukt 147,8 kg/h eines Gemisches aus der Säure, dem Formamid und rd. 0,9 kg Wasser gewonnen wurde.

Dieses Gemisch wurde in einer zweiten Glockenbodenkolonne, die 20 theoretische Böden hatte, bei 133 mbar und einem Rücklaufverhältnis von 1 in

18,8 kg/h eines Gemisches aus 17,9 kg/h Ameisensäure und 0,9 kg/h Wasser als Kopffraktion und
129,0 kg/h Di-n-propylformamid als Sumpffraktion, die in den Hydrolysereaktor zurückgeführt wurde,

zerlegt.

Somit wurden pro Stunde 18,8 kg 95 gew.%ige wäßrige Ameisensäure erhalten, entsprechend einer Ausbeute von 95 %. Etwa 5 % der Ameisensäure gingen infolge Zersetzung verloren.

## Beispiel 2

Auf die in Beispiel 1 beschriebene Weise, jedoch mit der gleichen molaren Menge N-Formylmorpholin anstelle des Di-n-propylformamids wurden pro Stunde 20,2 kg 95 gew.%ige wäßrige Ameisensäure, entsprechend einer Ausbeute von 95 %, erhalten. Der Methylformiat-Umsatz im Hydrolysereaktor betrug 44 %.

## Beispiel 3

In einem Rührreaktor wurden 61,8 kg/h (1 kmol/h) Methylformiat (eingesetzt als Gemisch mit 1,8 kg/h Methanol) mit 18 kg/h Wasser (1 kmol/h) bei 110 °C, etwa 8 bar und einer mittleren Verweilzeit von 20 min hydrolysiert, wobei die Hydrolyserate 29 % betrug.

Das Hydrolysegemisch wurde anschließend in einem zweiten Reaktor in einer durchschnittlichen Verweilzeit von 1 h bei 110 °C und etwa 6 bar mit 129,0 kg/h (1 kmol/h) Di-n-propylformamid versetzt, wobei die Hydrolyserate auf 41 % anstieg.

Die Aufarbeitung analog Beispiel 1 lieferte 19,9 kg/h einer 95 gew.%igen wäßrigen Ameisensäure, entsprechend einer Ausbeute von 100 %. Eine Zersetzung der Ameisensäure wurde hierbei nicht beobachtet.

**Patentansprüche**

1. Verfahren zur Herstellung von wasserfreier oder weitgehend wasserfreier Ameisensäure durch Hydrolyse von Methylformiat in Gegenwart eines Formamids und anschließende destillative Gewinnung der Ameisensäure aus dem Hydrolysegemisch, dadurch gekennzeichnet, daß man die Hydrolyse in Gegenwart von 0,5-3 mol pro Mol Methylformiat eines wasserlöslichen Formamides der allgemeinen Formel I

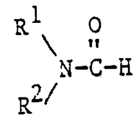

(I)

vornimmt, in welcher $R^1$ und $R^2$ Alkylgruppen oder gemeinsam unter Ausbildung eines 5- bis 7-gliedrigen Ringes eines Alkylengruppe mit der Maßgabe bedeuten, daß die Summe der C-Atome der Reste $R^1$ und $R^2$ 5 oder 6 beträgt und daß im Falle einer Alkylengruppe ein C-Atom, das nicht unmittelbar mit dem N-Atom verbunden ist, durch ein Sauerstoffatom ersetzt sein kann.

2. Verfahren nach Anspruch 1, dadurch gekennzeichnet, daß man die Hydrolyse in einer ersten Stufe ohne die Mitverwendung nennenswerter Mengen des Formamides I bis zur Gleichgewichtseinstellung führt und daß man sie zur Erhöhung des Umsatzes in einer zweiten Stufe in Gegenwart der definitionsgemäßen Mengen eines Formamides I, bezogen auf die Menge des ursprünglich eingesetzten Methylformiates, vornimmt.

3. Verfahren nach den Ansprüchen 1 und 2, dadurch gekennzeichnet, daß man als wasserlösliches Formamid I Di-n-propylformamid verwendet.

4. Verfahren nach den Ansprüchen 1 und 2, dadurch gekennzeichnet, daß man als wasserlösliches Formamid I N-3-Oxapentamethylenformamid (N-Formylmorpholin) verwendet.

**Claims**

1. A process for the preparation of anhydrous or substantially anhydrous formic acid by hydrolyzing methyl formate in the presence of a formamide and then obtaining the formic acid from the hydrolysis mixture by distillation, wherein the hydrolysis is carried out in the presence of 0.5-3 moles, per mole of methyl formate, of a water-soluble formamide of the formula I

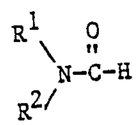

(I)

where $R^1$ and $R^2$ are each alkyl or together form an alkylene group, giving a 5-membered to 7-membered ring, with the proviso that the sum of the carbon atoms in $R^1$ and $R^2$ is 5 or 6 and that, in the case of an alkylene group, a carbon atom which is not directly bonded to the N atom can be replaced by an oxygen atom.

2. A process as claimed in claim 1, wherein the hydrolysis is carried out, in a first stage, in the absence of significant amounts of the formamide I until the equilibrium is established, and, in a second stage, in order to increase the conversion, is effected in the presence of a formamide I in the amounts conforming to the definition and based on the amount of methyl formate initially used.

3. A process as claimed in claims 1 and 2, wherein the water-soluble formamide I used is di-n-propylformamide.

4. A process as claimed in claims 1 and 2, wherein the water-soluble formamide I used is N-3-oxapentamethylene formamide (N-formylmorpholine).

**Revendications**

1. Procédé de préparation d'acide formique anhydre ou largement anhydre, par hydrolyse de formiate de méthyle en présence d'un formamide, suivie de récupération par distillation de l'acide formique à partir du mélange d'hydrolyse, caractérisé en ce qu'on procède à l'hydrolyse en présence de 0,5 à 3 mol, par mol de formiate de méthyle, d'un formamide hydrosoluble de formule générale I

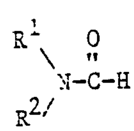

(I)

dans laquelle $R^1$ et $R^2$ représentent des groupes alkyle ou constituent ensemble, avec formation d'un noyau penta- à heptagonal, un groupe alkylène, avec ces conditions que la somme des atomes de C des radicaux $R^1$ et $R^2$ s'élève à 5 ou 6 et que dans le cas d'un groupe alkylène, un atome de C, qui n'est pas lié

directement à l'atome de N, puisse être remplacé par un atome d'oxygène.

2. Procédé selon la revendication 1, caractérisé en ce qu'on conduit l'hydrolyse, dans un premier temps, sans utilisation simultanée de quantités appréciables du formamide I, jusqu'à l'établissement de l'équilibre, et en ce que dans un second temps, pour élever le degré de transformation, on la conduit en présence des quantités conformes à la définition d'un formamide I, sur la base de la quantité du formiate de méthyle mis en réaction initialement.

3. Procédé selon la revendication 1 ou 2, caractérisé en ce qu'on utilise, comme formamide hydrosoluble I, le di-n-propylformamide.

4. Procédé selon la revendicatiön 1 ou 2, caractérisé en ce qu'on utilise, comme formamide hydrosoluble I, le N-3-oxapentaméthylèneformamide (N-formylmorpholine).